# EUROPÄISCHE PATENTSCHRIFT

(11) **EP 1 869 450 B1**
(45) Veröffentlichungstag und Bekanntmachung des Hinweises auf die Patenterteilung: **12.11.2008**
(21) Anmeldenummer: 06723634.9
(22) Anmeldetag: 22.03.2006
(51) Int. Cl.: G01N 33/48

(54) **VERFAHREN ZUR VERHINDERUNG DER ZEITABHÄNGIGEN RNA-EXPRESSION IN BIOLOGISCHEN ZELLEN**
METHOD FOR PREVENTING TIME-DEPENDENT RNA-EXPRESSION IN BIOLOGICAL CELLS
PROCEDE POUR EMPECHER L'EXPRESSION EN FONCTION DU TEMPS DANS DES CELLULES BIOLOGIQUES

(30) Priorität: 22.03.2005 DE 102005013261
(43) Veröffentlichungstag der Anmeldung: 26.12.2007
(73) Patentinhaber: Adnagen AG, 30853 Hannover-Langenhagen (DE)
(72) Erfinder: ZIEGLSCHMID, Veit, 21442 Toppenstedt (DE); BÖCHER, Oliver, 8952 Schlieren-Zürich (CH); ALBERT, Winfried, 82377 Penzberg (DE)
(74) Vertreter: Pfenning, Meinig & Partner GbR
(86) Internationale Anmeldenummer: PCT/EP2006/002642
(87) Internationale Veröffentlichungsnummer: WO 2006/100063

(56) Entgegenhaltungen:
- WO-A-94/07532
- WO-A-02/056030
- NOCENTINI S ET AL: "Survival, DNA synthesis and ribosomal RNA transcription in monkey kidney cells treated by formaldehyde." MUTATION RESEARCH. APR 1980, Bd. 70, Nr. 2, April 1980 (1980-04), Seiten 231-234, XP002392272 ISSN: 0027-5107
- RAINEN L ET AL: "Stabilization of mRNA Expression in Whole Blood Samples" CLINICAL CHEMISTRY, AMERICAN ASSOCIATION FOR CLINICAL CHEMISTRY, WASHINGTON, DC, US, Bd. 48, Nr. 11, November 2002 (2002-11), Seiten 1883-1890, XP002273125 ISSN: 0009-9147
- BAECHLER E C ET AL: "Expression levels for many genes in human peripheral blood cells are highly sensitive to ex vivo incubation" GENES AND IMMUNITY, Bd. 5, Nr. 5, Juli 2004 (2004-07), Seiten 347-353, XP002392273 ISSN: 1466-4879

### SEQUENZPROTOKOLL

<110> Adnagen AG
<120> Reagenz und Verfahren zur Verhinderung der zeitabhängigen Expression in biologischen Zellen
<130> Adnagen 060431
<140> 060431
   <141> 2006-03-22
<150> DE 10 2005 013 261.8
   <151> 2006-03-22
<160> 8
<170> PatentIn Ver. 2.1
<210> 1
   <211> 24
   <212> DNA
   <213> Künstliche Sequenz
<220>
   <223> Beschreibung der künstlichen Sequenz: Oligonukleotid
<400> 1
   aatcgtcaat gccagtgtac ttca 24
<210> 2
   <211> 24
   <212> DNA
   <213> Künstliche Sequenz
<220>
   <223> Beschreibung der künstlichen Sequenz: Oligonukleotid
<400> 2
   taacgcgttg tgatctcctt ctga 24
<210> 3
   <211> 24
   <212> DNA
   <213> Künstliche Sequenz
<220>
   <223> Beschreibung der künstlichen Sequenz: Oligonukleotid
<400> 3
   tcagcttcta ctctggtgca caac 24
<210> 4
   <211> 24
   <212> DNA
   <213> Künstliche Sequenz
<220>
   <223> Beschreibung der künstlichen Sequenz: Oligonukleotid
<400> 4
   tggtagtagt cggtgctggg atct 24
<210> 5
   <211> 24
   <212> DNA
   <213> Künstliche Sequenz
<220>
   <223> Beschreibung der künstlichen Sequenz: Oligonukleotid
<400> 5
   cccagtgtgt caactgcagc cagt 24
<210> 6
   <211> 24
   <212> DNA
   <213> Künstliche Sequenz
<220>
   <223> Beschreibung der künstlichen Sequenz: Oligonukleotid
<400> 6
   cagatgggca tgtaggagag gtca 24
<210> 7
   <211> 24
   <212> DNA
   <213> Künstliche Sequenz
<220>
   <223> Beschreibung der künstlichen Sequenz: Oligonukleotid
<400> 7
   ctggagaaga gctacgagct gcct 24
<210> 8
   <211> 24
   <212> DNA
   <213> Künstliche Sequenz
<220>
   <223> Beschreibung der künstlichen Sequenz: Oligonukleotid
<400> 8
   acaggactcc atgcccagga agga 24

## Beschreibung

Die vorliegende Erfindung betrifft ein Reagenz zur Verhinderung der zeitabhängigen, induzierten Expression in biologischen Zellen in einer Probe. Sie betrifft gleichermaßen ein Verfahren hierzu sowie derart behandelte Zellpräparate.

Derartige Reagenzien und derartige Verfahren werden insbesondere im Bereich der molekular-biologischen Analyse und der medizinischen Diagnostik, insbesondere der Tumordiagnostik, benötigt.

Viele Nachweisverfahren zielen darauf, die Genexpression auf mRNA-Ebene zu analysieren. Dabei können exprimierte Gene als Nachweis spezieller Zellen gewertet werden. Es kann aber auch das Expressionsmuster aller untersuchten Gene genutzt werden, um Rückschlüsse über den Zustand einer Zelle zu ziehen oder um im Vergleich von zwei biologischen Proben deren Unterschiede zu charakterisieren.

Es wurde beschrieben, dass es zwischen der Probennahme und der Probeanalyse zum Abbau transkribierter mRNA in der Probe aufgrund von Änderungen in den Umgebungsbedingungen der Probe im Anschluss an die Probeentnahme kommen kann (Becker S. et al., 2004 Clin Chem 50 (4), 785-786).

Überraschenderweise tritt aber ein anderes schwerwiegendes Problem bei den in der Anmeldung beschriebenen Verfahren zum Nachweis von zellassoziierter mRNA auf, und zwar eine zeitabhängige illegitime Expression von mRNA ex vivo (ex vivo = außerhalb des menschlichen oder tierischen Körpers). Es kann nach der Probennahme die Expression neuer mRNA induziert werden. Diese zeitabhängig induzierte Expression verfälscht das Analyseergebnis. Die vorliegende Erfindung beschäftigt sich mit diesem Problem.

Eine derartige illegitime, zeitabhängig induzierte Expression wird bereits in einer Vielzahl von Publikationen beschrieben, beispielsweise in Baechler et al., Genes Immun. Band 5, Seite 347 bis 353 (2004).

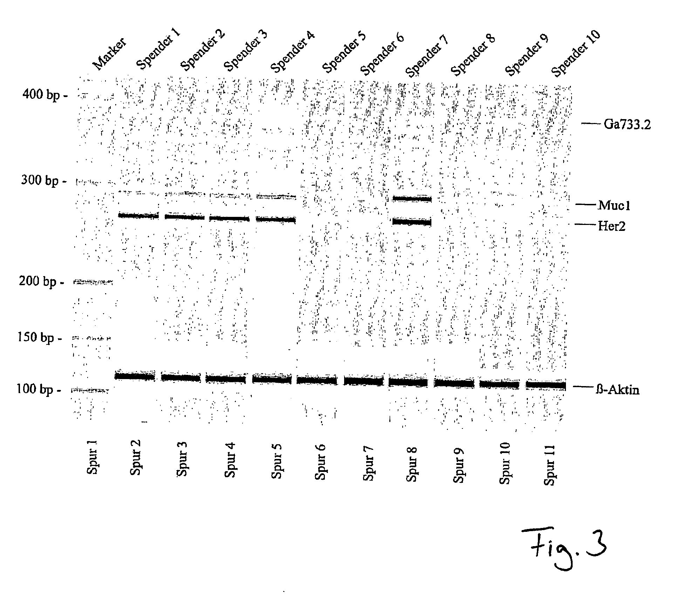

Neuerdings sind hochsensitive Analyseverfahren entwickelt worden, beispielsweise wie in der EP 02 732 726 A1 beschrieben. Der Nachweis von Tumorzellen im menschlichen Blut nach dem Verfahren der EP 02 732 726 ist abhängig davon, dass der Expressionsstatus zum Zeitpunkt der Blutentnahme bis zur Analyse aufrechterhalten bleibt. Mit dem in der EP 02 732 726 beschriebenen Verfahren werden Tumorzellen über mindestens zwei Antikörper immunomagnetisch angereichert, deren mRNA wird isoliert und die Expression von mindestens zwei tumorassoziierten mRNA-Markern wird untersucht. Bereits nach wenigen Stunden (24 h-Wert gezeigt in Figur 3) werden jedoch ohne spezielle Behandlung der Blutproben Transkripte nachgewiesen, die zum Zeitpunkt der Blutentnahme nicht vorhanden waren. Diese illegitime Transkription führt zu einem falsch-positiven Ergebnis.

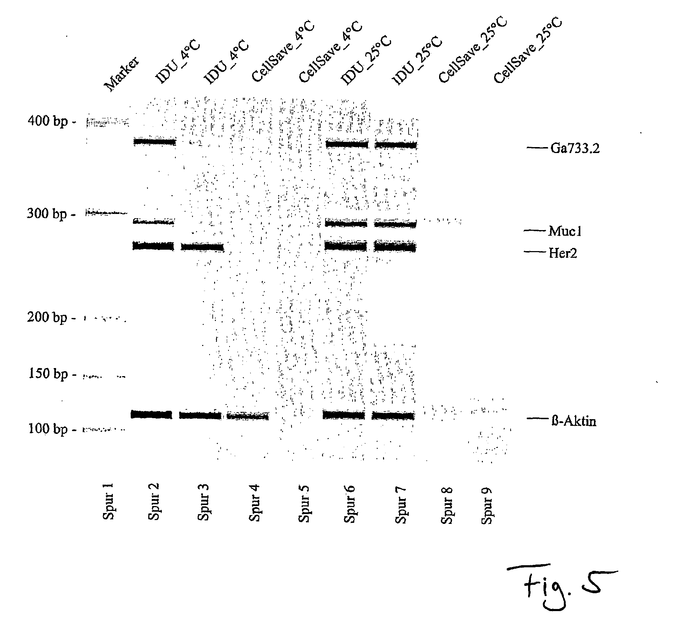

Beschriebene Verfahren, die darauf abzielen, lediglich den Abbau vorhandener mRNA zu verhindern sowie die Morphologie und Antigenstruktur von Zellen zu stabilisieren, sind nicht geeignet, um mit dem Verfahren der EP 02 732 726 kombiniert zu werden. Getestet wurden beispielsweise die Reagenzien CellSave^{®} (Immunicon Corporation, Huntingdon Valley, PA, USA) und Cyto-Chex^{®} (Streck Laboratories, Omaha, NE, USA), mit denen Proben nach Herstellerangaben gegen den Abbau von mRNA stabilisiert wurden. Dabei hat sich jedoch gezeigt, dass damit der Nachweis von Tumorzellen nach dem Verfahren der EP 02 732 726 nicht mehr möglich ist, da keine aussagekräftigen Ergebnisse erzielt werden können (siehe Figur 5). Das Problem der zeitabhängig induzierten Expression ist demnach mit den bekannten Verfahren nicht gelöst.

Die vorliegende Erfindung macht es sich also zur Aufgabe, ein Reagenz sowie ein Verfahren zur Verhinderung und Reduzierung der zeitabhängig induzierten Expression in biologischen Zellen in einer Probe sowie deren behandelte Zellpräparate zur Verfügung zu stellen.

Diese Aufgabe wird durch das Reagenz nach Anspruch 1, das Zellpräparat nach Anspruch 9 sowie das Verfahren nach Anspruch 15 gelöst. Vorteilhafte Weiterbildungen des Reagenzes, des Zellpräparates sowie des Verfahrens zur Verhinderung der zeitabhängig induzierten Expression werden in den jeweiligen abhängigen Ansprüchen gegeben.

Die vorliegende Erfindung beschäftigt sich also erstmals erfolgreich mit der Unterbindung der mRNA-Neusynthese (zeitabhängige, induzierte Expression). Diese besitzt bei Einsatz hochsensitiver, moderner Analysemethoden eine mindestens ebenso große Bedeutung wie die bisher betrachtete Stabilisierung der Oberflächenantigene oder der mRNS der Zielzellen. Nur so ist es möglich, die Zielzellen anhand ihrer mRNA-Expression unzweideutig zu charakterisieren.

Überraschenderweise wurde gefunden, dass die Zugabe eines Formaldehyddonors in sehr geringer Konzentration zu einer biologischen Probe, nämlich in einer Konzentration ≤ 0,075 % (w/v), vorteilhafterweise < 0,045 % (w/v), vorteilhafterweise ≤ 0,025 % (w/v) zu einer Verhinderung der zeitabhängigen illegitimen Expression führt. Als Formaldehyddonor kann dabei Imidazolidinyl-Harnstoff (IDU), Diazolidinyl-Harnstoff (DU), Dimethylol-5,5-Dimethylhydantoin, Dimethylol-Harnstoff, 2-Brom-2-Nitropropan-1,3-Diol (Bronopol), 1,3-bis(hydroxymethyl)-5,5-dimethylimidazolidine-2,4-dione (DMDM-Hydantoin), N5-Methyl-Tetrahydrofolsäure, N10-Methyl-Tetra-hydrofolsäure oder eine Mischung hiervon eingesetzt werden.

Mit dem erfindungsgemäßen Reagenz sowie dem erfindungsgemäßen Verfahren ist es daher erstmals möglich, die zeitabhängig induzierte Expression in biologischen Zellen in einer Probe ex vivo zu verhindern sowie eine Konservierung disseminierter Tumorzellen in Blutproben von Krebspatienten zu ermöglichen.

Hierzu wird nach Blutabnahme das Reagenz, gegebenenfalls gelöst in phosphatgepufferter Kochsalzlösung (PBS), sowie unter Zugabe des Gerinnungshemmers EDTA in der angegebenen Endkonzentration sofort mit dem Blut gemischt. Die Probe kann dann über einen Zeitraum von mehreren Tagen, beispielsweise bis zu mindestens 72 h bei 4 °C gelagert werden, ohne dass illegitime Expression innerhalb der Probe auftritt. Weiterhin können der Probe weitere zellstabilisierende Substanzen, wie z.B. Polyethylenglycol (PEG) und/oder Proteaseinhibitoren zugesetzt werden.

Um die erfindungsgemäße Wirkung zu demonstrieren, wurde in den folgenden Beispielen der Effekt der erfindungsgemäßen Zusätze auf die Spezifität eines Tumorzellennachweises mittels Analyse von Blutproben gesunder Spender sowie von Tumorpatienten untersucht. Außerdem wurde der Effekt der erfindungsgemäßen Zusätze auf die Sensitivität des Tumorzellnachweises mittels Analyse an Blutproben gesunder Spender mit inokulierten Tumorzellen untersucht. Die Analyse erfolgt dabei mittels vorgeschalteter Zellselektionierung und anschließender Expressionsprofilanalyse, wie sie in der europäischen Patentanmeldung EP 02 732 726 beschrieben sind. Diese europäische Patentanmeldung wird bezüglich des dort offenbarten Analyseverfahrens vollumfänglich in die vorliegende Anmeldung aufgenommen.

Desweiteren wurde die klinische Sensitivität mittels Analyse stabilisierter Blutproben von Brust- und Colorektal-Krebspatienten ermittelt. Alle untersuchten Patienten befanden sich in einem fortgeschrittenen Krankheitsstadium (M1) und wurden mit palliativer Therapie behandelt. Die erfindungsgemäßen Zusätze wurden unmittelbar nach Abnahme der Blutproben mit den Blutproben vermischt und sofort, nach 24 h, 48 h und nach 72 h Lagerung bei 4°C analysiert.

Es ist dabei zu erwähnen, dass sich die folgenden Beispiele sämtlich zwar auf Blutproben beziehen, jedoch das erfindungsgemäße Verfahren auch geeignet ist, um andere Zellsuspensionen oder Gewebsproben zu untersuchen. Zellsuspensionen können dabei Körperflüssigkeiten oder auch in Suspension gebrachte Gewebezellen, z.B. Gewebestücke, Gewebeschnitte und dergleichen, sein.

Es zeigen
- Figur 1: den Nachweis zeitabhängig induzierter Transkription in IDU behandelten Blutproben 24 h nach Abnehmen;
- Figur 2: den Nachweis zeitabhängig induzierter Transkription in IDU behandelten Blutproben 48 h nach Abnahme;
- Figur 3: den Nachweis zeitabhängig induzierter Transkription in nicht-behandelten Blutproben;
- Figur 4: die Erfassung von 2 Brusttumorzellen in mit IDU behandelten Blutproben;
- Figur 5: die Erfassung von Brusttumorzellen in mit verschiedenen Systemen behandelten Blutproben;
- Figur 6: typische Beispiele für den Nachweis disseminierter Tumorzellen in IDU behandelten Blutproben von Brustkrebspatientinnen;
- Figur 7: den Nachweis zeitabhängig induzierter Transkription in mit DU behandelten Blutproben;
- Figur 8: den Nachweis zeitabhängig induzierter Transkription in mit Bronopol behandelten Blutproben.

Bei sämtlichen im Folgenden dargestellten Beispielen wurde eine Analyse, wie sie in der EP 02 732 726 beschrieben ist, durchgeführt. Dabei wurden zur Zellselektion Antikörper an Magnetpartikel gekoppelt. Als Antikörper wurden dabei die im Folgenden in Tabelle 1 dargestellten Antikörper verwendet.

**Tabelle 1**

| **Antigen** | **Klon** | **Firma** |
|---|---|---|
| Epitheliales Membran Antigen | GP1.4 | Novocastra |
| Epitheliales Antigen | Ber-EP4 | DAKO |
| Muc 1 | HMPV | Pharmingen |

Die Magnetpartikel wurden dabei in einer Konzentration von 4 x 10⁸ Beads/ml (CELLection^{™} Pan Mouse IgG Kit, Firma Dynal) verwendet. Die Verhältnisse zwischen der Antikörperkonzentration und den daran gekoppelten Antikörpern sind in Tabelle 2 wiedergegeben.

**Tabelle 2**

| **Klon** | **Antikörperkonzentration** | **µl Antkörper/25µl** **Partikel** |
|---|---|---|
| BerEP4 | 0,32 mg/ml | 4 µl |
| HMPV | 0,5 mg/ml | 4 µl |
| GP1.4 | 0,2 mg/ml | 0,3 µl |

Die so vorbereiteten Magnetpartikel wurden zu gleichen Teilen gemischt und in 100 µl PBS je 5 ml Probe dem Blut (EDTA) zugegeben.

Nach 30 min Inkubation im Überkopfschüttler wurden die Magnetpartikel, die gegebenenfalls als Zell-Antikörper-Magnetpartikelkomplexe vorlagen, mittels eines Magnetpartikelkonzentrators (MPC^{®}-S, Firma Dynal) 3mal mit PBS gewaschen und die anhaftenden Zellen anschließend entsprechend des im folgenden beschriebenen mRNS-Isolierungsprotokolls behandelt.

Die mRNS-Isolierung erfolgte mittels Oligo(dT)-gekoppelter Magnetpartikel, Dynabeads^{®} mRNA Direct^{™} Micro Kit (Firma Dynal). Diese Isolierung erfolgte entsprechend der in dem Kit angegebenen Herstellerangaben.

An die Isolierung der mRNS schließt sich eine reverse Transkription an, bei der die mRNS in cDNS umgeschrieben wird.

**Tabelle 3: Komponenten der cDNS-Synthese**

| Die cDNS-Synthese erfolgt in einem 20 µl-Reaktionsansatz | |
|---|---|
| **Komponenten** | **Volumen** |
| mRNS | 29,5 µl |
| 10 x RT-Puffer | 4 µl |
| dNTP-Mix | 4 µl |
| RNase-Inhibitor (RNAsin, Promega) | 0,5 µl |
| Reverse Transkriptase | 2 µl |

Die cDNS-Synthese (Tabelle 3) erfolgte bei 37 °C für eine Stunde mit nachfolgender Inaktivierung der reversen Transkriptase durch Erhitzen für 5 Minuten bei 95 °C und anschließender Abkühlung auf Eis. Hierzu wurde ein Sensiskript Reverse Transkriptase Kit, Firma Qiagen, Hilden nach dort angegebenen Protokollen verwendet.

Anschließend an die Umschreibung der mRNS in cDNS erfolgte eine Polymerase-Kettenreaktion (PCR) mit ß-Aktin als interner Kontrolle.

Dabei wurden die in Tabelle 4 aufgeführten Oligonukleotide als PCR-Primer zur Amplifikation von cDNS verwendet.

**Tabelle 4: Liste der PCR-Primer**

| Primername | Sequenz 5' -> 3' | PCR-Produkt |
|---|---|---|
| Tumormarker | | |
| GA733.2 sense | AATCGTCAATGCCAGTGTACTTCA | 383 bp |
| GA733.2 sense | TAACGCGTTGTGATCTCCTTCTGA | |
| CA15-3 sense | TCAGCTTCTACTCTGGTGCACAAC | 293 bp |
| CA-15-3 antisense | TGGTAGTAGTCGGTGCTGGGATCT | |
| Her-2 sense | CCCAGTGTGTCAACTGCAGCCAGT | 270 bp |
| Her-2 antisense | CAGATGGGCATGTAGGAGAGGTCA | |

| Interne Kontrolle | | |
|---|---|---|
| Aktin sense | CTGGAGAAGAGCTACGAGCTGCCT | 114 bp |
| Aktin antisense | ACAGGACTCCATGCCCAGGAAGGA | |

Die PCR wurde mit dem in Tabelle 5 angegebenen Ansatz durchgeführt.

**Tabelle 5: PCR-Ansatz**

| Die PCR-Synthese erfolgte in einem 50 µl-Reaktionsansatz | |
|---|---|
| Komponenten | Volumen |
| cDNS | 8 µl |
| Primer | 4 µl |
| HotStarTaq | 25 µl |
| H₂O | 13 µl |

Die PCR-Bedingungen (Zyklenzahl, Zyklenführung etc.) sind in der Tabelle 6 gegeben; Temperaturänderungen erfolgten mit 2 °C/s.

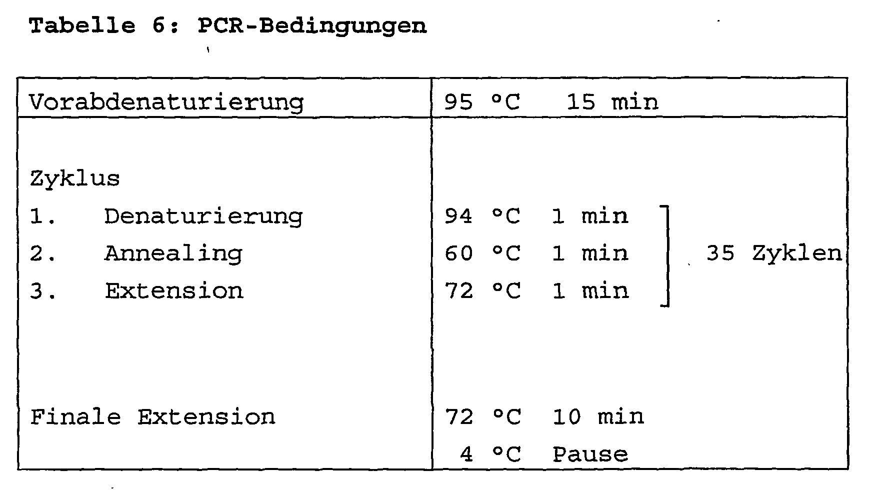

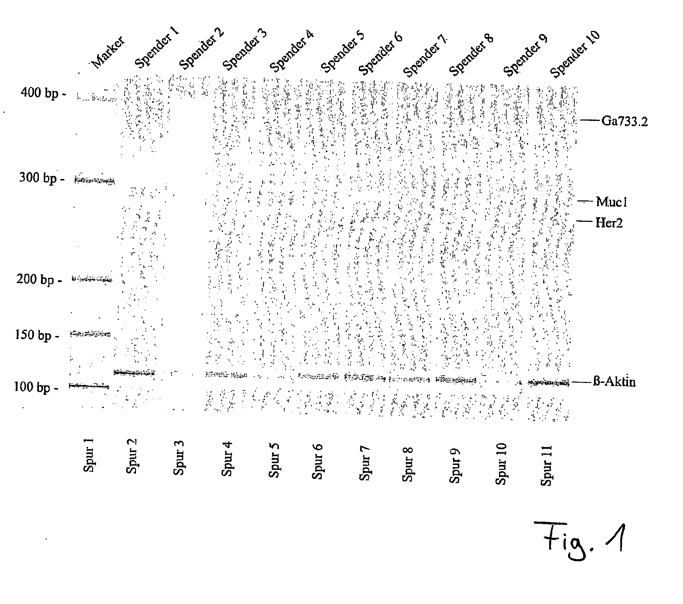

Die so erzeugten Amplifikate der cDNS wurden elektrophoretisch aufgetrennt mittels eines Bioanalyzer 2100 (Firma Agilent). Hierzu wurde 1 µl des PCR-Produktes in dem Bioanalyzer auf einem DNS-Chip 1000 aufgetrennt und das Trennergebnis elektronisch dokumentiert. Das Analyseergebnis wird für positiv befunden, wenn die Bandenintensität mindestens eines Tumormarkers > 6,7 ist. Der Bandenintensität entspricht die Einheit "Peakhöhe" des Agilents. Peaks mit einer Höhe von < 4,0 werden negativ bewertet. Peaks, deren Höhe von 4,0 bis 6,7 liegt, können weder als positiv noch als negativ bewertet werden. Auf diese Weise wurden die Figuren 1 bis 6 erzeugt und ausgewertet.

Figur 1 zeigt den Nachweis zeitabhängig induzierter Transkription in mit IDU behandelten Blutproben.

Spur 1 zeigt dabei eine Leiter mit Größenmarkern, Spuren 2 bis 11 zeigen die bei zehn verschiedenen gesunden Spendern nachzuweisenden amplifizierten DNS-Fragmente der tumorassoziierten Transkripte und einer internen Kontrolle von ß-Aktin. Die Position der jeweiligen Banden für ß-Aktin (Actin) und die drei tumorassoziierten Transkripte GA733-2 (384 bp), MUC-1 (292 bp) und Her-2 (265 bp) sind in Fig. 1 bezeichnet.

Die Proben der zehn gesunden Spender wurden dabei mit 0,045 % (w/v) IDU versetzt und anschließend bei 4 °C für 24 h gelagert. Die amplifizierten DNA-Fragmente wurden nach Analyse der Blutproben mittels Hochspannungskapillar-Gelelektrophorese in einem Bioanalyzer 2100 (Agilent Technologies) nachgewiesen. Es ist zu erkennen, dass die Analyse bei allen zehn gesunden Spendern (Spender 1 bis Spender 10 in Spuren 2 bis 11) negativ ausgefallen ist (Peakhöhen unter Schwellenwert). D.h. es kann keinerlei illegitime zeitabhängig induzierte Expression nachgewiesen werden.

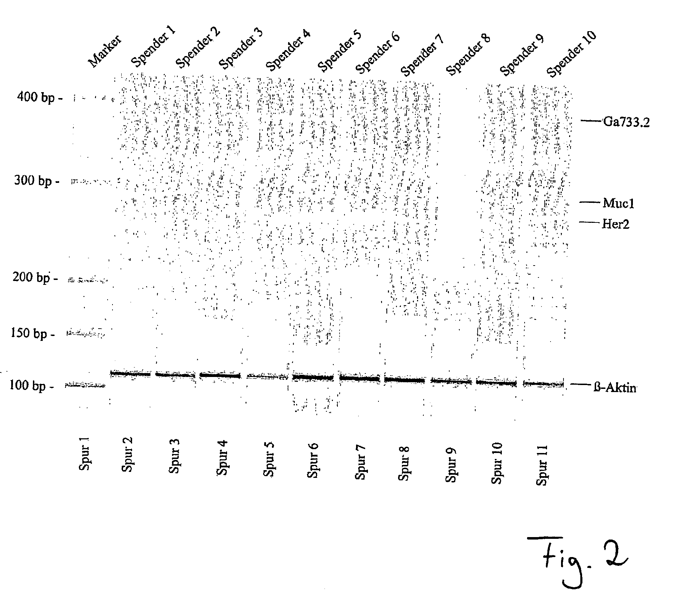

Figur 2 zeigt dieselbe Untersuchung, nun jedoch nach Lagerung der Proben der zehn gesunden Spender für 48 h. Auch hier konnte noch immer keine zeitabhängige Expression tumorassoziierter Transkripte nachgewiesen werden.

Figur 3 zeigt demgegenüber dieselben Blutproben wie in Figur 1 und in Figur 2, jedoch ohne Zugabe von 0,025 % (w/v) IDU. Es ist zu erkennen, dass bei diesen nicht behandelten Blutproben illegitime Expression der tumorassoziierten Transkripte auftrat.

Mit den Untersuchungen gemäß den Figuren 1 bis 3 wurde eindeutig nachgewiesen, dass mit den angegebenen Konzentrationen eines Formaldehyddonors wirksam die illegitime zeitabhängig induzierte Expression in Zellen innerhalb eines Zellpräparats unterdrückt werden kann. Die erfindungsgemäßen Zusätze erhalten eine hohe Spezifität.

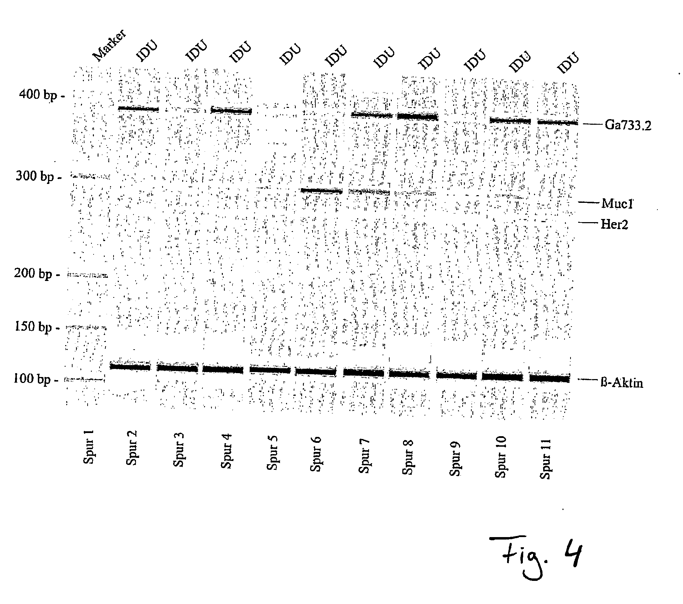

Figur 4 zeigt demgegenüber die Erfassung von Brusttumorzellen in mit 0,045 % (w/v) IDU behandelten Blutproben zehn gesunder Spender (Spuren 2 bis 10). In diese Proben wurden je 5 ml Blut zwei Brustkrebszellen (MCF7) inokuliert. Die dargestellte Untersuchung auf das Expressionsmuster der tumorassoziierten Transkripte Her-2, MUC-1 und GA733.2 wurde nach einer Lagerung der Blutproben bei 4°C über 48 h durchgeführt. Alle weiteren Analyseparameter entsprechen denjenigen in Figuren 1 bis 3. Es ist zu erkennen, dass die tumorassoziierten Transkripte Her-2, MUC-1 und GA733.2, die über die Inokulierung von MCF7-Brustkrebszellen in die Blutproben eingebracht wurden, eindeutig nachgewiesen werden. Figur 4 zeigt damit eindeutig, dass auch so geringe Zellzahlen, wie 2 Zellen/5 ml Blut noch nach 48 h nachgewiesen werden können, wenn die Blutprobe erfindungsgemäß behandelt wurde, ohne den Nachweis durch illegitime zeitabhängig induzierte Expression zu behindern. Ein Verlust an Sensitivität des angestrebten Tumorzellennachweises wird also durch das erfindungsgemäße Verfahren ebenfalls verhindert.

Figur 5 zeigt Vergleichsversuche mit der marktüblichen Stabilisierungslösung "CellSave" von Immunicon. Hierzu wurden in je 5 ml Blut gesunder Spender jeweils 5 Brustkrebszellen (HCC1954) inokuliert. Zwei der Proben wurden mit 0,025 % (w/v) IDU erfindungsgemäß behandelt und bei 4 °C gelagert (Spuren 2 und 3). Zwei weitere Proben wurden in entsprechender Weise behandelt, jedoch bei 25 °C gelagert (Spuren 6 und 7). Zwei weitere Proben wurden gemäß den Herstellerangaben mit dem Produkt CellSave stabilisiert und bei 4°C gelagert (Spuren 4 und 5). Die letzten beiden Proben in Figur 5 (Spuren 8 und 9) wurden ebenfalls gemäß den Herstellerangaben mit CellSave stabilisiert und bei 25 °C gelagert.

Wie zu erkennen ist, wird die Expression der Tumormarker Her-2, MUC-1 und GA733.2 in den inokulierten Brustkrebszellen sowie des Markers ß-Aktin in den erfindungsgemäß behandelten Proben (Spuren 2, 3, 6, 7) erfasst. Mit der käuflichen Stabilisierungslösung CellSave stabilisierte Proben (Spuren 4, 5, 8, 9) war ein sensitiver Nachweis weder des Markers ß-Aktin noch der Tumormarker Her-2, MUC-1 und Ga733.2 möglich.

Dies zeigt, dass im Vergleich zum Stand der Technik durch die vorliegende erfindungsgemäße Behandlung eine sehr effektive Erhaltung des Expressionsmusters in einer biologischen Probe erzielt wird, die erlaubt, die nachgeschaltete Analyse hoch selektiv und sensitiv durchzuführen.

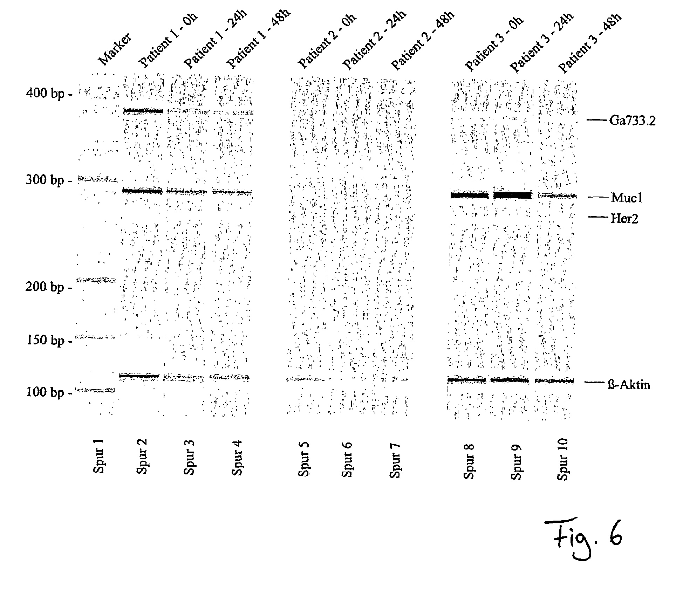

Figur 6 zeigt die Analyse von Blutproben von Brustkrebspatienten unmittelbar nach der Blutprobenentnahme, nach 24 h sowie nach 48 h. Sämtliche dieser Proben wurden unmittelbar nach Blutabnahme mit 0,025 % (w/v) IDU versetzt und bei 4 °C gelagert.

Figur 6 zeigt in den Spuren 2 bis 4 die Untersuchungsergebnisse der Blutprobe eines Patienten unmittelbar nach der Blutentnahme, nach 24 h und 48 h nach der Blutentnahme. Es zeigt sich, dass das Expressionsmuster über die Zeit weitgehend gleich bleibt und folglich die IDU-Zugabe zu einer Bewahrung des Expressionsmusters führt. Entscheidend ist dabei, dass auch keinerlei illegitime zeitabhängig induzierte Expression auftritt. Dies ist auch bei der Probe des zweiten Patienten, die in den Spuren 5 bis 7 über die Zeit verfolgt wurde, der Fall. Dieser weist offensichtlich keine zirkulierenden Tumorzellen in der Blutprobe auf. Eine zeitabhängig induzierte Expression wurde ebenfalls nicht festgestellt. Auch die Probe des Patienten 3 in den Spuren 8 bis 10 zeigt, dass mit der Behandlung durch 0,025 % (w/v) IDU eine Bewahrung des Expressionsmusters über 48 h erzielt werden konnte.

Zusammenfassend lässt sich feststellen, dass der Tumorzellennachweis in mit 0,025 % (w/v) IDU behandelten Blutproben von Brustkrebspatientinnen sofort nach Abnahme, nach 24 h und nach 48 h einheitliche Ergebnisse zeigt, d.h. Blutproben, in denen zum Zeitpunkt der Abnahme Tumorzellen nachgewiesen werden konnten, zeigen auch nach 24 h und 48 h einen positiven Tumorzellennachweis, während Proben ohne Tumorzellen auch nach 24 und 48 h negativ blieben, d.h. keine falsch-positiven Resultate entstehen, die durch eine zeitabhängige Expression ex vivo erzeugt sein könnten. Die untersuchten Blutproben zeigen also über einen Zeitraum von 48 h hinweg ein konstantes Expressionsmuster, d.h. Proben, die anfangs negativ waren, blieben negativ und positive Proben blieben positiv.

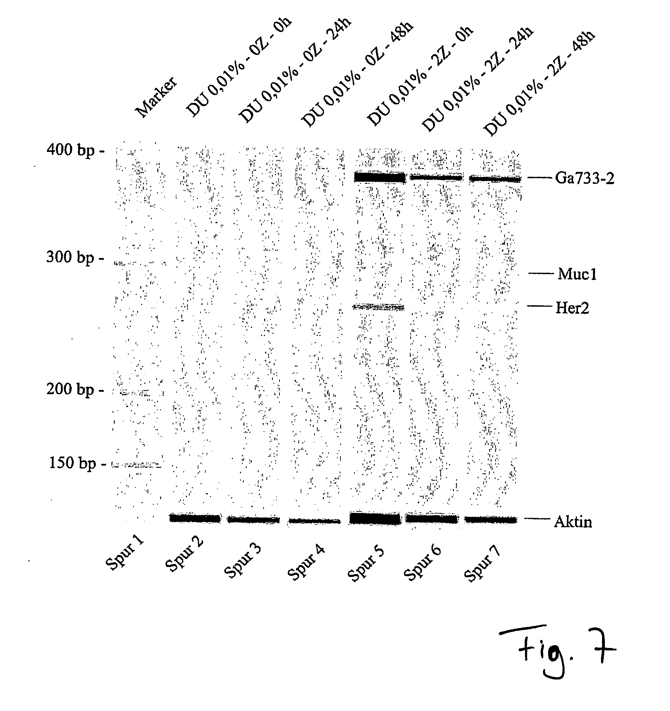

Figur 7 zeigt den Nachweis zeitabhängig induzierter Transkription in mit DU behandelten Blutproben. Der Nachweis erfolgte nach den zu IDU beschriebenen Protokollen.

Spur 1 zeigt dabei eine Leiter mit Größenmarkern, Spuren 2 bis 4 zeigen die bei einem gesunden Spender nachzuweisenden amplifizierten DNS-Fragmente der tumorassoziierten Transkripte und einer internen Kontrolle von ß-Aktin. Spuren 5 bis 7 zeigen die Transkripte von Proben mit 2 inokulierten Zellen der Tumorzelllinie Calu-3 zu den Zeitpunkten 0 h, 24 h und 48 h. Die Position der jeweiligen Banden für ß-Aktin (Actin) und die drei tumorassoziierten Transkripte GA733-2 (384 bp), MUC-1 (292 bp) und Her-2 (265 bp) sind in Fig. 7 bezeichnet.

Die Proben wurden dabei mit 2 inokulierten Calu-3-Zellen mit 0,01 % (w/v) DU versetzt und anschließend bei 4 °C für maximal 48 h gelagert. Die amplifizierten DNA-Fragmente wurden nach Analyse der Blutproben mittels Hochspannungskapillar-Gelelektrophorese in einem Bioanalyzer 2100 (Agilent Technologies) nachgewiesen. Es ist zu erkennen, dass die Analyse bei allen Proben ohne inokulierte Zellen (Spuren 2 bis 4) negativ ausgefallen ist (Peakhöhen unter Schwellenwert). D.h. es kann keinerlei illegitime zeitabhängig induzierte Expression nachgewiesen werden.

An den Spuren 5 bis 7 ist zu erkennen, dass tumorassoziierte Transkripte, die über die Inokulierung von Calu-3-Krebszellen in die Blutproben eingebracht wurden, eindeutig nachgewiesen werden. Figur 7 zeigt damit eindeutig, dass auch geringe Zellzahlen noch nach 48 h nachgewiesen werden können, wenn die Blutprobe erfindungsgemäß behandelt wurde, ohne den Nachweis durch illegitime zeitabhängig induzierte Expression zu behindern. Ein Verlust an Sensitivität des angestrebten Tumorzellennachweises wird also durch das erfindungsgemäße Verfahren ebenfalls verhindert.

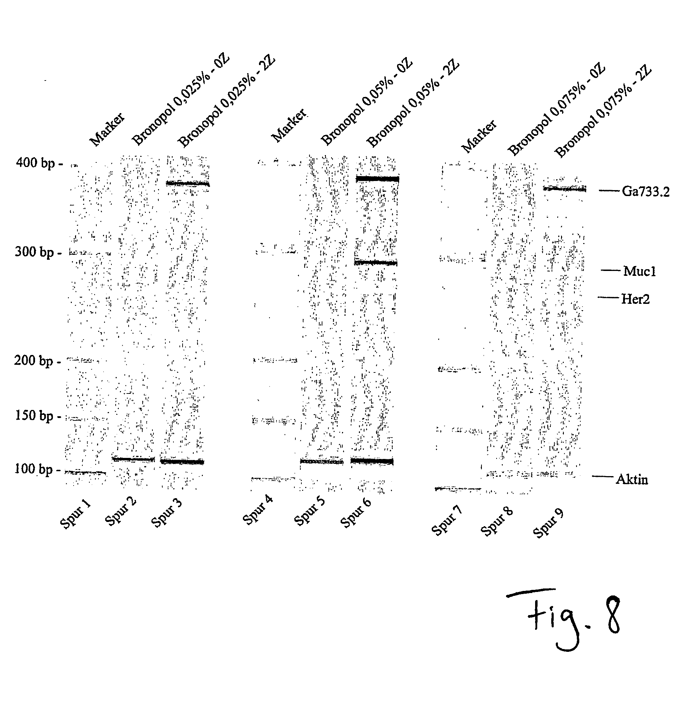

Figur 8 zeigt den Nachweis zeitabhängig induzierter Transkription in mit Bronopol behandelten Blutproben (2-Bromo-2-Nitropropan-1,3-Diol).

Spuren 1, 4 und 7 zeigen dabei eine Leiter mit Größenmarkern, Spuren 2, 5 und 8 zeigen die bei einem gesunden Spender nachzuweisenden amplifizierten DNS-Fragmente der tumorassoziierten Transkripte und einer internen Kontrolle von ß-Aktin. Die Spuren 3, 6 und 9 zeigen die Transkripte von Proben mit 2 inokulierten Zellen der Tumorzelllinie Calu-3. Die Position der jeweiligen Banden für ß-Aktin (Actin) und die drei tumorassoziierten Transkripte GA733-2 (384 bp), MUC-1 (292 bp) und Her-2 (265 bp) sind in Fig. 8 bezeichnet.

Die Proben eines gesunden Spenders wurden dabei mit 0,025 % (Spuren 2 und 3), 0,05 % (Spuren 5 und 6) und 0,075 % (w/v) (Spuren 8 und 9) Bronopol versetzt und anschließend bei 4 °C für 24 h gelagert. Die amplifizierten DNA-Fragmente wurden nach Analyse der Blutproben mittels Hochspannungskapillar-Gelelektrophorese in einem Bioanalyzer 2100 (Agilent Technologies) nachgewiesen. Es ist zu erkennen, dass die Analyse bei allen drei Konzentrationen Bronopol (Spuren 2, 5 und 8) negativ ausgefallen ist (Peakhöhen unter Schwellenwert). D.h. es kann keinerlei illegitime zeitabhängig induzierte Expression nachgewiesen werden. Die Spuren 3, 6 und 9 zeigen, dass tumorassoziierte Transkripte, die über die Inokulierung von Calu-3-Krebszellen in die Blutproben eingebracht wurden, eindeutig nachgewiesen werden. Figur 8 zeigt damit eindeutig, dass auch so geringe Zellzahlen, wie 2 Zellen, noch nach 24 h nachgewiesen werden können, wenn die Blutprobe erfindungsgemäß behandelt wurde, ohne den Nachweis durch illegitime zeitabhängig induzierte Expression zu behindern. Ein Verlust an Sensitivität des angestrebten Tumorzellennachweises wird also durch das erfindungsgemäße Verfahren ebenfalls verhindert.

Die oben dargestellten Versuchsergebnisse zeigen eindeutig sowohl eine hohe Effektivität als auch Verlässlichkeit des erfindungsgemäßen Reagenzes und des erfindungsgemäßen Verfahrens bei der Behandlung von Zellpräparaten, insbesondere von disseminierten Tumorzellen im Blut von Brustkrebspatientinnen. Bei Colorektal-Krebspatienten wurden vergleichbare Ergebnisse erzielt.

Die vorliegenden Versuchsergebnisse zeigen also, dass eine Verhinderung einer zeitabhängig induzierten Expression in biologischen Zellen in einer Probe ex vivo erzielt wird. Weiterhin konnten disseminierte Tumorzellen in peripherem Blut von Brust- und Colorektal-Krebspatienten durch die Zugabe der erfindungsgemäßen Reagenzien nachgewiesen werden. Es wurde keine zeitabhängig induzierte Transkription über 48 h nachgewiesen. In nicht-behandelten Blutproben wurde jedoch eine sehr starke zeitabhängig induzierte Expression schon nach 24 h beobachtet.

Die erfindungsgemäßen Reagenzien und das erfindungsgemäße Verfahren ermöglichen also eine Verhinderung einer zeitabhängig induzierten Expression in biologischen Zellen in einer Probe (insbesondere ex vivo) und gleichzeitig eine Aufbewahrung disseminierter Tumorzellen in peripherem Blut von Krebspatienten. Sie besitzen daher einen hohen logistischen und diagnostischen Wert, da hiermit auch ein längerer Transport und ein größerer zeitlicher Abstand zwischen Probenentnahme und Probenanalyse ermöglicht wird. Die erfindungsgemäßen Reagenzien und das erfindungsgemäße Verfahren eignen sich daher zum Einsatz in Blutabnahmesystemen.

## Patentansprüche

1. Verfahren zur Verhinderung der zeitabhängigen illegitimen Expression von mRNA in biologischen Zellen in einer Probe ex vivo,
**dadurch gekennzeichnet, dass** die Probe mit einem Reagenz, das einen Formaldehyddonor enthält, sowie gegebenenfalls einer Flüssigkeit vermischt wird.

2. Verfahren nach dem vorhergehenden Anspruch, **dadurch gekennzeichnet, dass** die Mischung des Reagenzes mit der Probe den Formaldehyddonor in einer Konzentration ≤ 0,075 % (w/v) enthält.

3. Verfahren nach dem vorhergehenden Anspruch, **dadurch gekennzeichnet, dass** die Konzentration des Formaldehyddonors in der Mischung des Reagenzes mit der Probe ≤ 0,045 % (w/v), vorteilhafterweise ≤ 0,025 % (w/v) beträgt.

4. Verfahren nach einem der drei vorhergehenden Ansprüche, **dadurch gekennzeichnet, dass** der Formaldehyddonor, Imidazolidinyl-Harnstoff, Diazolidinyl-Harnstoff, Dimethylol-5,5-Dimethylhydantoin, Dimethylol-Harnstoff, 2-Brom-2-Nitropropan-1,3-Diol, 1,3-bis(hydroxymethyl)-5,5-dimethylimidazolidine-2,4-dione (DMDM-Hydantoin), N5-Methyl-Tetrahydrofolsäure, N10-Methyl-Tetrahydrofolsäure oder eine Mischung hiervon enthält oder daraus besteht.

5. Verfahren nach einem der Ansprüche 1 bis 4, **dadurch gekennzeichnet, dass** die Flüssigkeit ein Puffer, physiologische Kochsalzlösung oder dergleichen ist.

6. Verfahren nach einem der Ansprüche 1 bis 5, **dadurch gekennzeichnet, dass** die damit behandelten biologischen Zellen antikörpervermittelt oder über andere gebräuchliche Verfahren separiert werden.

7. Verwendung eines Verfahrens nach einem der vorhergehenden Ansprüche, um die zeitabhängige Expression von mRNA in einer Probe mit biologischen Zellen zu verhindern, insbesondere in einer Körperflüssigkeit, Blut, Liquor, Aszites, Urin oder in einem Gewebestück oder einer Suspension, die Zellen aus Gewebeproben enthält, insbesondere zum Nachweis von hierin enthaltenen Tumorzellen.

## Claims

1. A method for preventing time-dependent illegitimate expression of mRNA in biological cells in an ex vivo sample,
**characterised in that**
the sample is mixed with a reagent which contains a formaldehyde donor and optionally with a liquid.

2. A method according to the preceding claim, **characterised in that** the mixture of the reagent with the sample contains the formaldehyde donor in a concentration ≤ 0.075% (wt./vol.).

3. A method according to the preceding claim, **characterised in that** the concentration of the formaldehyde donor in the mixture of the reagent with the sample amounts to ≤ 0.045% (wt./vol.), advantageously to ≤ 0.025% (wt./vol.).

4. A method according to any one of the three preceding claims, **characterised in that** the formaldehyde donor contains or consists of imidazolidinyl urea, diazolidinyl urea, dimethylol-5,5-dimethylhydantoin, dimethylol urea, 2-bromo-2-nitropropane-1,3-diol, 1,3-bis(hydroxymethyl)-5,5-dimethylimidazolidine-2,4-dione (DMDM hydantoin), N5-methyltetrahydrofolic acid, N10-methyltetrahydrofolic acid or a mixture thereof.

5. A method according to any one of claims 1 to 4, **characterised in that** the liquid is a buffer, physiological saline or the like.

6. A method according to any one of claims 1 to 5, **characterised in that** the biological cells treated therewith are separated by an antibody-mediated or other conventional method.

7. Use of a method according to any one of the preceding claims in order to prevent time-dependent expression of mRNA in a sample comprising biological cells, in particular in a bodily fluid, blood, liquor, ascites, urine or in a fragment of tissue or a suspension which contains cells from tissue samples, in particular for the detection of tumour cells contained therein.

## Revendications

1. Procédé pour empêcher l'expression illégitime d'ARNm en fonction du temps dans des cellules biologiques dans un échantillon ex vivo, **caractérisé en ce que** l'on mélange l'échantillon à un réactif, qui continent un donneur de formaldéhyde et, éventuellement, à un liquide.

2. Procédé selon la revendication précédente, **caractérisé en ce que** le mélange comprenant le réactif et l'échantillon contient le donneur de formaldéhyde en concentration ≤ 0,075 % (p/v).

3. Procédé selon la revendication précédente, **caractérisé en ce que** la concentration du donneur de formaldéhyde dans le mélange comprenant le réactif et l'échantillon est ≤ 0,045 % (p/v), de manière avantageuse ≤ 0,025 % (p/v).

4. Procédé selon l'une quelconque des trois revendications précédentes, **caractérisé en ce que** le donneur de formaldéhyde contient ou se compose des substances suivantes : imidazolidinyle-urée, diazolidinyle-urée, diméthylol-5,5-diméthylhydantoïne, diméthylol-urée, 2-bromo-2-nitropropan-1,3-diol, 1,3-bis(hydroxyméthyl)-5,5-diméthylimidazolidine-2,4-dione (DMDM-hydantoïne), acide N5-méthyl-tétrahydrofolique, acide N10-méthyl-tétrahydrofolique ou un mélange de celles-ci.

5. Procédé selon l'une quelconque des revendications 1 à 4, **caractérisé en ce que** le liquide est une solution tampon, une solution physiologique de sel de cuisine ou similaire.

6. Procédé selon l'une quelconque des revendications 1 à 5, **caractérisé en ce que** les cellules biologiques traitées de la sorte sont séparées par un procédé dépendant d'anticorps ou par d'autres procédés utiles.

7. Utilisation d'un procédé selon l'une quelconque des revendications précédentes, pour empêcher l'expression d'ARNm en fonction du temps dans un échantillon contenant des cellules biologiques, en particulier, dans un liquide corporel, le sang, le liquide céphalo-rachidien, l'ascite, l'urine ou dans un fragment de tissu ou dans une suspension, qui contient des cellules provenant d'échantillons tissulaires, en particulier, pour détection des cellules tumorales qui y sont contenues.
